# FASCICULE DE BREVET EUROPEEN

(11) **EP 3 319 667 B1**
(45) Date de publication et mention de la délivrance du brevet: **10.04.2019**
(21) Numéro de dépôt: 16736209.4
(22) Date de dépôt: 08.07.2016
(51) Int. Cl.: A61M 5/315

(54) **SERINGUE ET SON PROCÉDÉ D'ASSEMBLAGE**
SPRITZE UND VERFAHREN ZUR MONTAGE DAVON
SYRINGE AND METHOD FOR ASSEMBLING IT

(30) Priorité: 08.07.2015 FR 1556463
(43) Date de publication de la demande: 16.05.2018
(73) Titulaire: Guerbet, 93420 Villepinte (FR)
(72) Inventeur: CACLIN, Jérôme, 69360 Saint Symphorien d'Ozon (FR)
(74) Mandataire: Lavoix
(86) Numéro de dépôt international: PCT/EP2016/066319
(87) Numéro de publication internationale: WO 2017/005914

(56) Documents cités:
- EP-A1- 2 801 382
- WO-A1-2014/082412
- WO-A1-2014/121307
- WO-A2-2006/121513
- WO-A2-2009/019673
- DE-A1-102007 014 418
- US-A- 5 803 918
- US-A1- 2014 276 592

## Description

La présente invention concerne une seringue, ainsi qu'un procédé d'assemblage d'une telle seringue.

L'invention concerne notamment les seringues en matériaux rigides à usage médical qui sont remplies par pression.

De manière classique, une seringue comprend un corps cylindrique délimitant une chambre dans laquelle un piston est reçu.

Une seringue peut être remplie par dépression, c'est-à-dire que l'utilisateur tire sur le piston de cette seringue pour aspirer manuellement un liquide. L'utilisateur peut doser sa force et arrêter de tirer le piston lorsqu'il arrive en fin de course, afin d'éviter de faire sortir le piston entièrement du corps de la seringue.

Une seringue peut aussi être remplie par pression, c'est-à-dire qu'elle est reliée à un contenant qui, lorsqu'une pression est exercée sur lui, amène le liquide dans la seringue. Il est bien souvent difficile de définir précisément la pression à exercer sur le contenant pour remplir comme on le souhaite la seringue et il y a ainsi un risque important qu'un excès de pression exercé sur le contenant fasse sortir entièrement le piston du corps de la seringue.

Dans le cas par exemple des procédures de chimio-embolisation (aussi appelée c-TACE en anglais pour « conventional Trans Arterial Chemo Embolization), la seringue est utilisée en combinaison avec un robinet médical et est remplie par pression, c'est-à-dire que la seringue est reliée à une deuxième seringue qui amène le liquide dans la première seringue. L'utilisateur ne manipule pas la première seringue, de sorte qu'en poussant sur le piston de la deuxième seringue, il ne se rend pas compte de l'effet de sa force sur la première seringue. Le piston de la première seringue risque alors d'être éjecté. La procédure doit alors être recommencée à zéro puisque les conditions de stérilité de celle-ci ne sont plus assurées. De plus, cela expose le praticien à des produits potentiellement dangereux, par exemple des produits cytotoxiques dans le cas de la chimio-embolisation.

US 5 803 918 divulgue une seringue dont le corps est équipé d'une bague de retenue externe comportant un doigt qui est recourbé dans la chambre du corps de la seringue. Le doigt assure le blocage du piston, l'empêchant d'être séparé du corps de la seringue. La surface de contact entre le piston et la bague de retenue est formée par l'extrémité du doigt, qui est de dimension réduite. Ainsi, l'efficacité du blocage du piston est limitée, puisque ce système donne uniquement un retour tactile qui indique au praticien quand s'arrêter de tirer sur le piston dans le cas d'un remplissage par dépression, mais ne l'avertit pas suffisamment dans le cas d'un remplissage par pression. Ce système n'empêche donc pas de sortir malencontreusement le piston du corps de la seringue.

Il y a donc un besoin d'avoir des seringues permettant d'empêcher leur piston de sortir entièrement de leur corps. Cette problématique interfère par ailleurs avec le fait que, sur le marché, il existe des seringues avec des corps en matière plastique déformable et des seringues avec des corps en matière plastique rigide donc non déformable.

Dès lors qu'on cherche à retenir le piston d'une seringue à corps en matière plastique rigide lorsque le piston est poussé vers la sortie du corps de la seringue, l'efficacité de la retenue est soit limitée en raison de la trop faible interférence de contact entre le piston et un dispositif solidarisé au corps de seringue, soit obtenue par des aménagements coûteux et complexes reposant sur un piston en plusieurs parties distinctes et/ou sur un assemblage initial compliqué entre le piston et le corps de seringue.

EP 2 801 382 divulgue une seringue avec un corps en matière plastique déformable puisque la tête du piston inséré dans cette seringue ne comporte aucun joint d'étanchéité. Dans cette seringue est inséré un dispositif, appelé « corps de fermeture », qui est non déformable et qui composé de deux parties reliées entre elles par, d'un côté, une charnière mince et par, de l'autre côté, un système de verrouillage attachant les deux parties l'une à l'autre une fois agencées autour du piston. Ce système de verrouillage empêche le dispositif de se déplier, c'est-à-dire qu'il empêche les deux parties de s'écarter l'une de l'autre, quand ce dispositif est inséré dans le corps de la seringue. La rétention du piston par ce dispositif résulte exclusivement de l'engagement transversal, dans des ouvertures latérales du corps de seringue, de deux clips saillants respectivement portés par les deux parties du dispositif : une telle disposition est peu performante d'un point de vue mécanique et ne permet pas d'empêcher l'éjection du piston hors du corps de seringue en cas de remplissage par pression avec une force non maitrisée.

WO 2014/121307 A1, qui peut être considéré comme l'état de la technique le plus proche de l'invention, divulgue une seringue dont le corps est pourvu fixement, à son extrémité d'où émerge le piston de la seringue, d'un insert qui est traversé par la tige du piston de manière à interférer avec l'insert en générant des cliquetis audibles lorsque le piston coulisse dans la chambre du corps. En pratique, la capacité effective de cet insert pour retenir le piston et l'empêcher de sortir entièrement du corps de la seringue est douteuse.

Un des buts de l'invention est donc de proposer une nouvelle seringue permettant de retenir de manière simple et performante le piston dans le corps de seringue lors de son utilisation.

A cet effet, l'invention a pour objet une seringue telle que définie à la revendication 1.

Grâce à l'invention, le piston est bloqué efficacement dans le corps de la seringue au moyen du dispositif de retenue, qui est assemblé facilement en étant monté en force dans le corps de la seringue et qui présente une surface de contact étendue avec le piston pour le retenir.

Des aspects avantageux mais non obligatoires de l'invention sont spécifiées aux revendications dépendantes.

Un autre aspect de l'invention concerne un procédé d'assemblage d'une seringue, tel que défini à la revendication 14. Une disposition optionnelle avantageuse est définie à la revendication 15.

L'invention sera mieux comprise et d'autres aspects de celle-ci apparaitront plus clairement à la lumière de la description qui va suivre d'une seringue et de son procédé d'assemblage, conformes à l'invention, donnée uniquement à titre d'exemple et faite en référence aux dessins dans lesquels :
- la figure 1 est une vue en perspective d'une seringue conforme à l'invention ;
- la figure 2 est une coupe longitudinale en perspective de la seringue de la figure 1 ;
- la figure 3 est une coupe longitudinale de la seringue de la figure 1 ;
- la figure 4 est une vue à plus grande échelle du détail IV à la figure 3 ;
- la figure 5 est une coupe selon la ligne V-V à la figure 4 ;
- la figure 6 est une vue en perspective d'un dispositif de retenue faisant partie de la seringue de la figure 1, dans une position libre ;
- la figure 7 est une vue de face du dispositif de retenue de la figure 6, dans une position de montage ;
- la figure 8 est une vue de face du dispositif de retenue de la figure 6, dans une position assemblée ;
- les figures 9 à 11 sont des vues en perspective d'un piston de la seringue de la figure 1 et du dispositif de retenue, dans trois configurations d'assemblage successives ; et
- les figures 12 à 17 sont des vues respectivement similaires aux figures 1, 3, 4, 6, 7 et 8, illustrant une variante de la seringue, conforme à l'invention.

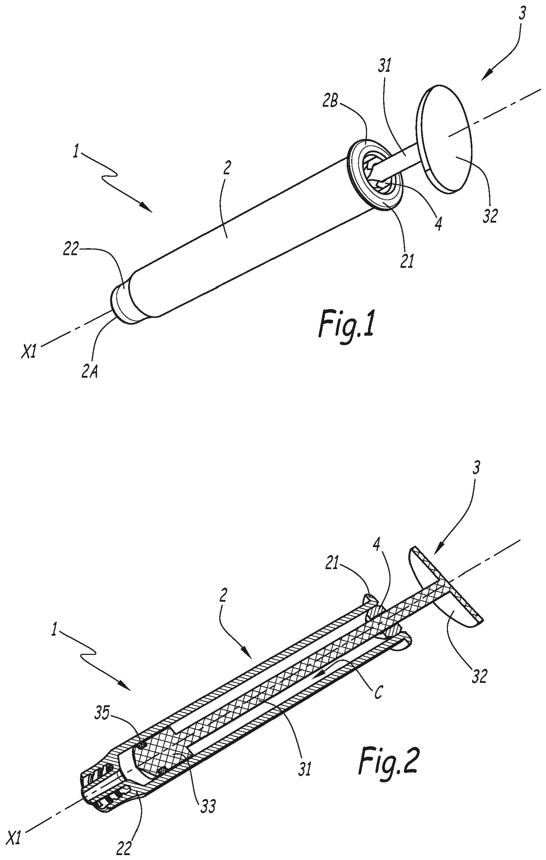

La seringue 1 représentée sur les figures 1 à 5 comprend un corps 2 de préférence cylindrique, un piston 3 et un dispositif de retenue 4. La seringue 1 s'étend en longueur le long d'un axe longitudinal X1, qui est un axe de révolution pour le piston 3 et le corps 2.

Le corps 2 est de forme tubulaire et délimite une chambre C dans laquelle le piston 3 est monté coulissant, le long de l'axe X1. On note D2 un diamètre intérieur du corps 2, égal au diamètre de la chambre C. De manière préférentielle, le piston 3 est monté en liaison pivot glissant, c'est-à-dire qu'en plus d'être coulissant, il est pivotant dans le corps 2 de la seringue 1. Une première tête 22 du corps 2 forme une première extrémité 2A du corps 2. La tête 22 est équipée d'un cône d'extrémité également appelé « cône Luer », prévu pour permettre la connexion de la seringue avec un autre dispositif, ce dernier dispositif répondant notamment à une des normes Luer usuelles, telles que ISO 594 et ISO 80369, sans que l'indication de ces normes ne soit limitative. A l'opposé de la tête 22, le long de l'axe X1, le corps 2 est pourvu d'une collerette externe annulaire 21 qui forme une deuxième extrémité 2B du corps 2. De manière préférentielle, la collerette externe annulaire 21 n'a pas vocation à servir à la préhension de la seringue. De préférence, la collerette externe annulaire 21 est en saillie radiale vers l'extérieur de moins de 5 mm, plus préférentiellement inférieure ou égale à 2 mm ou encore plus préférentiellement strictement inférieure à 2 mm.

Le piston 3 comporte une tige 31, de diamètre D31, s'étendant le long de l'axe X1. Une première extrémité longitudinale 3A du piston 3 est formée par une deuxième tête 33, de diamètre D33 strictement supérieur au diamètre D31 de la tige 31. La tête 33 comporte préférentiellement une gorge 34 annulaire périphérique, dans laquelle un élément d'étanchéité 35 est disposé. De manière préférentielle, l'élément d'étanchéité 35 est choisi parmi au moins un joint torique et un joint à lèvre. Le joint à lèvre est choisi parmi un joint double-lèvre et un joint triple-lèvre. De manière encore plus préférentielle, l'élément d'étanchéité 35 est un joint torique. L'élément d'étanchéité 35 peut être constitué de plusieurs joints toriques, par exemple deux joints toriques.

En variante, l'élément d'étanchéité 35 fait partie intégrante de la tête 33 du piston 3, il est réalisé par exemple par injection, en même temps que le piston 3, ou par surmoulage. Dans ce mode de réalisation, l'élément d'étanchéité se présente alors sous la forme d'une ou plusieurs protubérances annulaires sur la surface de la tête 33 du piston 33.

Un appuie-doigt 32 forme une deuxième extrémité longitudinale 3B du piston 3, à l'opposé de la tête 33.

Globalement, l'appuie-doigt 32 s'étend en travers de l'axe X1. En projection dans un plan géométrique perpendiculaire à l'axe X1, l'appuie-doigt 32 présente un contour périphérique indifférent, par exemple de forme ronde, elliptique, circulaire, rectangulaire ou carrée. Préférentiellement, le contour périphérique précité de l'appuie-doigt est de forme elliptique. On note D32 la plus petite dimension transversale de l'appuie-doigt 32, c'est-à-dire sa dimension minimale dans un plan géométrique perpendiculaire à l'axe X1.

De manière générale, l'appuie-doigt 32 est, sur sa face opposée axialement à la tige 31, plat, convexe ou concave. De manière préférentielle, l'appuie-doigt 32 est convexe ou concave. Dans un mode particulier de réalisation, lorsque la seringue 1 a une chambre C de volume inférieur ou égal à 3 mL, l'appuie-doigt 32 est convexe. Dans un autre mode particulier de réalisation, lorsque la seringue 1 a une chambre C de volume strictement supérieur à 3 mL et inférieur ou égal à 15 mL, l'appuie-doigt 32 est convexe. Dans un autre mode particulier de réalisation, lorsque la seringue 1 a une chambre C de volume strictement supérieur à 15 mL, l'appuie-doigt 32 est concave. Dans un autre mode de réalisation de l'invention, lorsque la seringue 1 a une chambre C de volume supérieur ou égal à 20 mL, l'appuie-doigt 32 est concave. Par appuie-doigt convexe, on entend préférentiellement au sens de la présente invention, que l'appuie-doigt suive la forme des doigts qui seront apposés sur lui. Par appuie-doigt concave, on entend préférentiellement au sens de la présente invention, que l'appuie-doigt suive la forme de la paume de la main qui sera apposée sur lui.

Le piston 3 est monobloc, autrement dit la tige 31, la tête 33 et l'appuie-doigt 32 sont en une seule et même pièce. Le piston 3, mis à part le ou les éléments d'étanchéité 35, ne résulte pas de l'assemblage de plusieurs éléments entre eux.

Le dispositif de retenue 4 a la forme d'un anneau ouvert, et il est déformable de manière à pouvoir être monté autour de la tige 31 du piston 3. De manière préférentielle, le dispositif de retenue 4 est déformable uniquement à son montage sur la tige 31 du piston 3. De manière préférentielle, le dispositif de retenue 4 est dépourvu d'aménagement le verrouillant en une forme figée d'anneau fermé lui-même, ce qui permet au dispositif de retenue 4 d'exercer, notamment par résilience structurelle, une force radiale vers l'extérieur du corps 2 de la seringue 1 une fois qu'il est inséré dans celui-ci.

Le dispositif de retenue 4 est monté autour du piston 3 qui est ensuite inséré dans la chambre C. Le dispositif de retenue 4 entourant le piston 3 est alors monté en force dans le corps 2, à l'intérieur de la chambre C, du côté de l'extrémité 2B du corps 2.

Le dispositif de retenue 4 est par exemple en matière plastique, et peut être fabriqué par toute technique connue de l'homme du métier, et notamment par injection ou par extrusion.

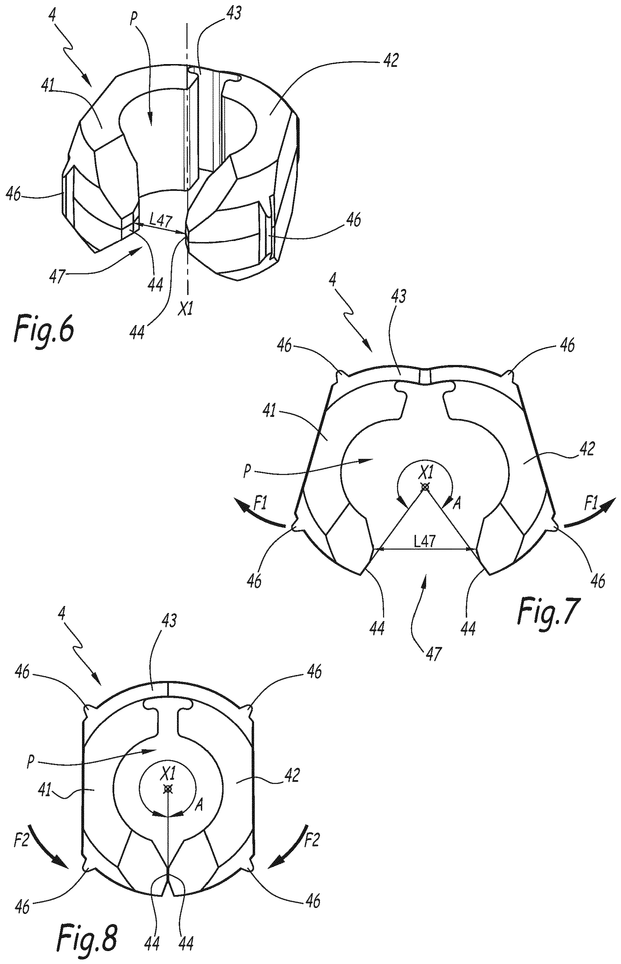

La figure 6 montre le dispositif de retenue 4 dans une position libre, c'est-à-dire lorsqu'aucune sollicitation mécanique extérieure ne lui est appliquée. La position libre est celle obtenue lors de l'injection plastique.

Le dispositif de retenue 4 comporte deux parties 41 et 42 en forme de C ou d'anneau partiel, qui délimitent entre elles un passage P destiné à recevoir la tige 31 du piston 3. Chaque partie 41 et 42 comporte une surface intérieure en forme de portion de cylindre, centrée sur l'axe X1 à un jeu fonctionnel près, de manière à définir une forme globalement cylindrique pour le passage P lorsque le dispositif de retenue 4 dans une position assemblée montrée à la figure 8.

Dans la position libre, des extrémités 44 de chaque partie 41 et 42 sont espacées entre elles d'une distance non nulle, de sorte que le dispositif de retenue 4 forme un anneau ouvert. Une ouverture 47 d'entrée dans le passage P est délimitée entre les extrémités 44 des parties 41 et 42. Les parties 41 et 42 sont raccordées entre elles par une zone amincie 43 déformable, suffisamment fine pour permettre à un utilisateur d'écarter ou de rapprocher les parties 41 et 42 l'une de l'autre.

De manière préférentielle, la zone amincie 43 a une épaisseur strictement supérieure à 0,6 mm, voire comprise entre 0,7 et 3 mm, voire comprise entre 0,7 et 2,5 mm ou entre 0,7 et 1 mm ou encore entre 2 et 2,5 mm. Dans un mode de réalisation particulier, lorsque la seringue 1 a un volume de chambre C inférieur ou égal à 3 mL, la zone amincie 43 déformable a une épaisseur entre 0,7 et 1 mm, préférentiellement de 0,7 mm. Dans un mode de réalisation particulier, lorsque la seringue 1 a un volume de chambre C supérieur ou égal à 20 mL, la zone amincie 43 déformable a une épaisseur entre 2 et 2,5 mm, préférentiellement de 2,2 mm,

Une telle épaisseur pour la zone amincie 43 induit un effet ressort entre les parties 41 et 42. De manière plus préférentielle, cet effet ressort se produit de façon durable, c'est-à-dire pendant toute la durée d'utilisation de la seringue 1, après le montage en force du dispositif de retenue 4. Ainsi, la zone amincie 43 déformable reste « nerveuse » une fois montée dans le corps 2 de la seringue. Une fois qu'elle a été sollicitée après le montage en force du dispositif de retenue 4 dans le corps 2 de la seringue, cette zone amincie 43 ne subit pas de déformation plastique irrémédiable, c'est-à-dire qui résulterait d'un dépassement de la limite élastique de cette zone amincie 43. L'effet ressort induit une force radiale spécifique permettant un maintien plus performant du dispositif de retenue à l'intérieur du corps de la seringue,

On note A un secteur angulaire centré sur l'axe X1, c'est-à-dire sur le centre géométrique du passage P, et délimité par les extrémités 44 du dispositif de retenue 4. Le secteur angulaire A s'étend de part et d'autre de l'ouverture 47.

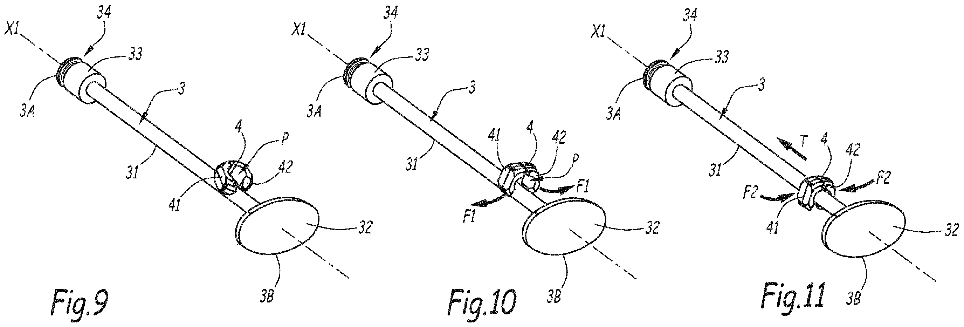

La figure 9 montre le dispositif de retenue 4 dans une première position de montage, dans laquelle il est amené contre la tige 31 du piston 3. L'ouverture 47 du passage P du dispositif de retenue 4 est positionnée contre la tige 31. La largeur L47 de l'ouverture 47, mesurée entre les extrémités 44 des parties 41 et 42, est inférieure au diamètre D31 de la tige 31 du piston 3.

Comme visible à la figure 10, pour permettre l'insertion de la tige 31 dans le passage P du dispositif de retenue 4, les parties 41 et 42 du dispositif de retenue 4 sont écartées l'une de l'autre, comme représenté par les flèches F1, de manière à agrandir l'ouverture 47 du passage P. Il est alors possible de positionner la tige 31 dans le passage P. Dans cette position du dispositif de retenue 4, représentée à la figure 7, la largeur L47 de l'ouverture 47 est supérieure ou égale au diamètre D31 de la tige 31.

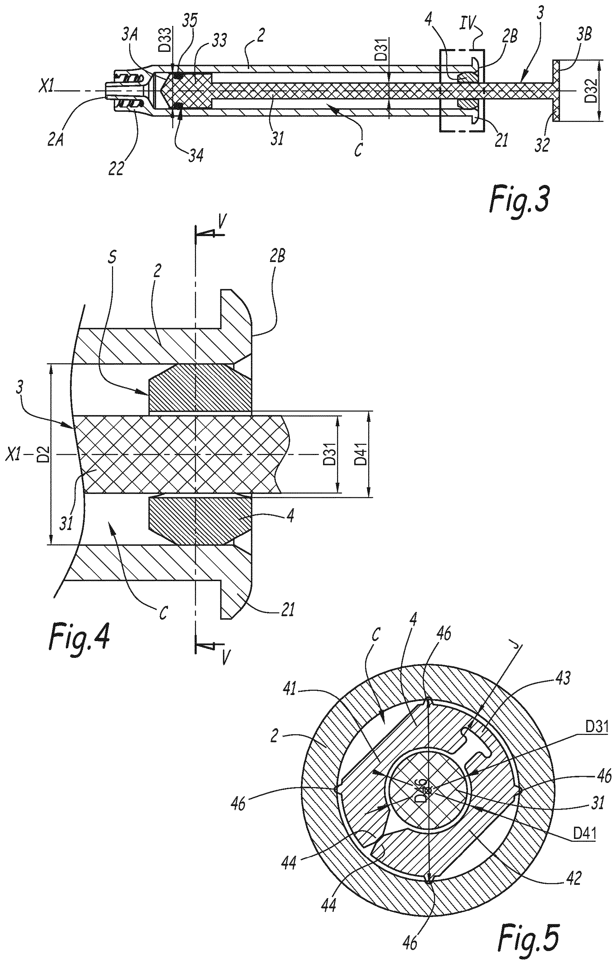

Puis, les parties 41 et 42 sont rapprochées l'une de l'autre, comme représenté par les flèches F2 à la figure 11, ce qui fait que la largeur L47 redevient inférieure au diamètre D31 de la tige 31. Le rapprochement des parties 41 et 42 est poursuivi jusqu'à passer le dispositif de retenue 4 en position assemblée. Dans cette position assemblée du dispositif de retenue tel que présenté à la figure 5, les extrémités 44 sont en contact l'une avec l'autre, refermant l'ouverture 47 du passage P, qui, comme évoqué plus haut, est alors de forme sensiblement cylindrique, comme visible à la figure 8. Le dispositif de retenue 4 entoure alors la tige 31 du piston sur 360°.

Dans le cadre de l'invention, dans la position assemblée, le secteur angulaire A est strictement supérieur à 180°, de préférence supérieur à 270°, de manière plus préférentielle, supérieur à 300°, de manière encore plus préférentielle, égal à 360°.

Ensuite, l'ensemble formé par le piston 3 et le dispositif de retenue 4 est monté en force dans le corps 2 de la seringue 1, par un mouvement de translation axiale T, c'est-à-dire coaxialement à l'axe X1.

Le dispositif de retenue 4 comporte des nervures 46 qui s'étendent en saillie parallèlement à l'axe X1. Autrement dit, chaque nervure 46 présente la forme d'une saillie allongée, dont la direction longitudinale est parallèle à l'axe X1. Chaque nervure s'étend ainsi en longueur entre les extrémités axiales opposées du dispositif de retenue 4, en particulier dans la région courante de ce dernier reliant ses deux extrémités axiales.

Dans l'exemple représenté, la première partie 41 porte de deux à quatre premières nervures 46, et la deuxième partie 42 porte de deux à quatre autres nervures 46. Ainsi, le dispositif de retenue 4 dénombre quatre à huit nervures. De manière préférentielle, lorsque la seringue 1 a un volume de chambre C inférieur ou égal à 3 mL, le dispositif de retenue 4 comporte deux nervures sur chaque partie 41 et 42, soit quatre nervures au total. De manière préférentielle, lorsque la seringue 1 a un volume de chambre C supérieur ou égal à 20 mL, le dispositif de retenue 4 comporte plus de deux nervures, plus préférentiellement quatre nervures sur chaque partie 41 et 42. Ainsi dans ce mode de réalisation, le dispositif de retenue 4 comporte plus de quatre nervures, préférentiellement huit nervures au total. Plus généralement, les nervures 46 sont disposées sur le dispositif de retenue 4 en étant de préférence réparties à distance les unes des autres autour de l'axe X1.

Au niveau de chaque nervure, une dimension extérieure maximale D46 transversale du dispositif de retenue 4 est strictement supérieure au diamètre D2 de la chambre C du corps 2. De manière préférentielle, la différence entre la dimension D46 et le diamètre D2 est choisie afin d'obtenir une force d'emmanchement suffisante pour retenir le piston 3 lors du remplissage de la seringue 1 par pression. La dimension D46 est radiale par rapport à l'axe X1.

En dehors des nervures 46, la dimension extérieure maximale transversale du dispositif de retenue 4 est strictement inférieure au diamètre D2 de la chambre C du corps 2, laissant apparaitre un jeu fonctionnel J.

Lors du montage en force du dispositif de retenue 4 dans la chambre C, les nervures 46 se déforment plastiquement. Leur écrasement correspondant permet de maintenir fermement le dispositif de retenue 4 dans le corps 2 de la seringue 1.

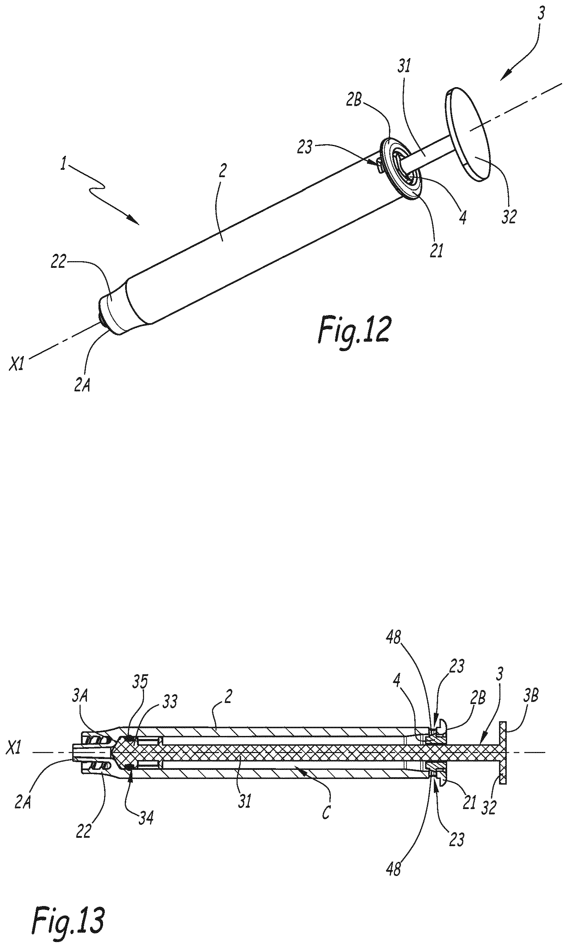

Selon la variante illustrée aux figures 12 à 17, le dispositif de retenue 4 comporte en outre au moins un clip 48 sur l'une ou l'autre des parties 41 et 42 du dispositif. De manière préférentielle, deux clips 28 sont prévus, respectivement sur la partie 41 et sur la partie 42 du dispositif de retenue 4. En position assemblée du dispositif de retenue 4, le ou chaque clip 48 est reçu dans un logement 23 prévu à cet effet dans le corps 2 de la seringue 1. Le ou chaque logement 23 débouche dans la chambre C du corps 2 de la seringue 1 et s'étend sur au moins une partie de l'épaisseur radiale de la paroi de ce corps 2. Préférentiellement, comme dans l'exemple considéré sur les figures 12 à 15, le ou chaque logement 23 traverse de part en part la paroi du corps 2 de la seringue 1.

Lors du montage en force du dispositif de retenue 4 dans la chambre C, le ou chaque clip 48 est engagé axialement en force à l'intérieur de la chambre C, en étant contraint radialement vers l'axe X1 par la paroi du corps 2 de la seringue 1, jusqu'à atteindre le ou l'un des logements 23 : par libération de contrainte, le clip 48 s'engage alors dans le logement 23 de manière transversale, voire radiale, à l'axe X1. Suivant une forme de réalisation préférentielle, mise en oeuvre dans l'exemple considéré sur les figures 12 à 15, le ou chaque clip 48 présente, en coupe dans un plan contenant l'axe X1, un profil externe convergeant vers la tête 22 du corps 2 de la seringue. Dans tous les cas, le ou les clips 48 viennent en renfort des nervures 46 pour améliorer le maintien dans le temps du dispositif de retenue 4 à l'intérieur du corps 2 de la seringue 1.

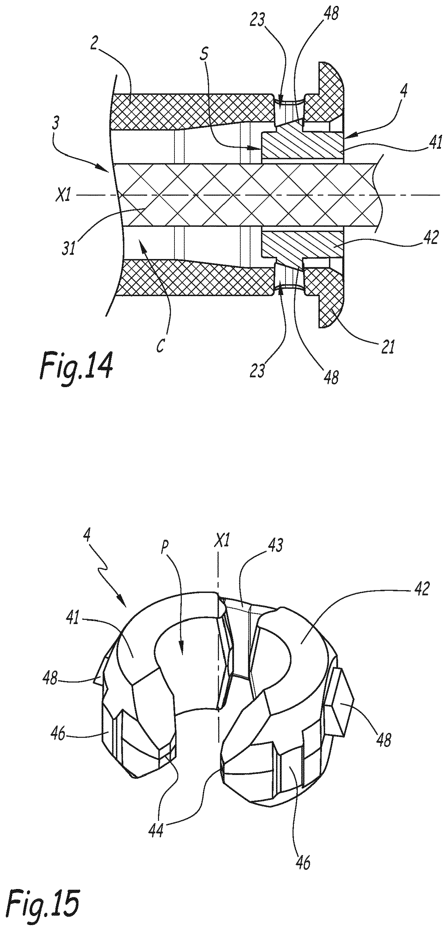

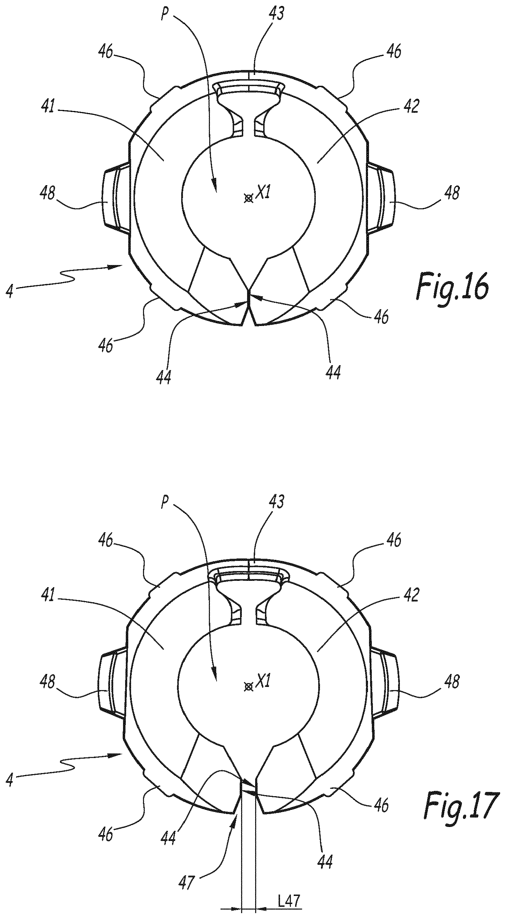

Par ailleurs, indépendamment des aménagements liés aux clips 48, le dispositif de retenue 4 tel que considéré aux figures 12 à 17 est tel que, dans la position assemblée de ce dispositif de retenue, les extrémités 44 de ses parties 41 et 42 ne sont pas en contact l'une avec l'autre, c'est-à-dire qu'elles sont espacées l'une de l'autre par une distance non nulle, valant par exemple quelques dixièmes de millimètres, comme visible sur la figure 17. Ainsi, dans la position assemblée du dispositif de retenue, le passage P, qui, comme évoqué plus haut, est alors de forme sensiblement cylindrique, a son ouverture 47 qui presque entièrement mais totalement refermée: comme indiqué sur la figure 17, la largeur L47 de cette ouverture 47 présente ainsi une valeur non nulle, qui, bien entendu, est inférieure au diamètre D31 de la tige 31 du piston 3. Avant d'atteindre cette position assemblée, autrement dit lors du montage en force du dispositif de retenue 4, les extrémités des parties 41 et 42 sont rapprochées l'une de l'autre, jusqu'à, le cas échéant, se toucher, comme illustré par la figure 16, ce qui facilite la mise en place du dispositif de retenue 4 à l'intérieur de la chambre C du corps 2 de la seringue 1. Le ré-écartement des extrémités 44 une fois le dispositif de retenue en position assemblée, résultant de la propension de ce dispositif à reprendre sa position libre de la figure 15, permet au dispositif de renforcer son maintien radial à l'intérieur du corps de la seringue.

Le corps 2 de la seringue 1 est dans un matériau plastique présentant un module de Young (appelé également « module d'élasticité » ou « module de rétractation mécanique ») strictement supérieur à 1600 MPa, préférentiellement supérieur ou égal à 1800 MPa, encore plus préférentiellement égal à 1900 MPa. Ce matériau plastique a comme propriété d'être rigide et donc de ne pas être déformable. De manière préférentielle, un matériau plastique présentant un tel module de Young est choisi parmi les copolymères méthyméthacrylate-acrylonitrile-butadiène styrène (MABS), les polycarbonates (PC), le polymétacrylate de méthyle (PMMA), les polyamides (PA), préférentiellement les PA11 et PA12, le polysulfone (PSU), les copolymères de cyclooléfines (COC) et les polymères de cyclooléfines (COP). Ces matériaux plastiques ont aussi comme avantage d'être transparents.

De manière préférentielle, le corps 2 et le dispositif de retenue 4 sont réalisés dans le même matériau. De manière plus préférentielle, le corps 2 et le dispositif de retenue 4 sont réalisés dans des matériaux permettant d'augmenter le frottement entre eux. De manière encore plus préférentielle, le corps 2 et le dispositif de retenue 4 sont réalisés dans un matériau et avec un état de surface augmentant les frottements entre ces deux pièces. Un exemple d'état de surface augmentant les frottements entre le corps 2 et le dispositif de retenue 4 est obtenu en faisant subir aux deux pièces un polissage « poli miroir ».

Le dispositif de retenue 4 comporte une surface S perpendiculaire à l'axe X1 et tournée vers la chambre C du corps 2, prévue pour venir en contact avec la tête 33 du piston 3 lorsque celui-ci est en position arrière, afin de l'empêcher de sortir entièrement du corps 2. La surface S a une forme d'anneau ouvert dans la position libre, et une forme d'anneau fermé dans la position assemblée.

Le passage P du dispositif de retenue 4 en position assemblée présente un diamètre D41 qui est strictement inférieur au diamètre D33 de la tête du piston 3. Plus la différence entre les diamètres D41 et D33 est importante, et plus la surface S est étendue. Ceci est favorable à la retenue du piston 3 dans la chambre C.

De préférence, le diamètre D33 est au moins supérieur de 10%, de préférence au moins supérieure de 25%, au diamètre D41.

Cette différence de diamètre est particulièrement avantageuse pour une seringue 1 dont la chambre C a un volume faible, de l'ordre de 1mL. De préférence, le volume de la chambre C est inférieur à 25 mL, de préférence inférieur ou égal à 20 mL, plus préférentiellement inférieur ou égal à 3 mL et encore plus préférentiellement inférieur ou égal à 1 mL.

L'invention permet de s'affranchir de toute relation entre le diamètre D41 du passage P du dispositif de retenue 4 en position assemblée, d'une part, et les diamètres D33 de la tête 33 du piston 3 et D32 de l'appuie-doigt 32, d'autre part. Ceci permet de maitriser la surface S du dispositif de retenue 4.

Dans les seringues de l'art antérieur, en particulier celles dont le corps est en matériau rigide, les fonctions de retenue du piston sont habituellement supportées par des structures créées directement dans le corps de la seringue. Ces structures devant tout de même permettre le passage du piston dans le corps de la seringue et le démoulage du corps lors de sa réalisation en contre-dépouille, elles ne remplissent pas suffisamment leur fonction de retenue.

En outre, le montage du dispositif de retenue 4 dans le corps 2 s'effectue sans nécessiter le passage d'un point dur, tel qu'obtenu par un bossage annulaire dans le corps de seringue, ce qui n'endommage pas le système d'étanchéité 35 du piston 3. En d'autres termes, la séquence d'assemblage de la seringue 1 n'altère par le système d'étanchéité 35 entre le corps 2 et la tête 33 du piston 3. Le dispositif de retenue 4 est assemblé après que le piston 3 soit monté dans le corps 2.

Le corps 2 de la seringue a une géométrie simple. Par un corps 2 à « géométrie simple », on entend au sens de la présente invention le fait que ce corps 2 ne présente pas de contre-dépouille. Le moule d'injection du corps 2 de la seringue 1 est ainsi simplifié et fiabilisé.

Par ailleurs, le dispositif de retenue 4 permet d'améliorer le guidage du piston 3 en position reculée, c'est-à-dire vers l'arrière par rapport à la tête de piston, ce qui contribue à garantir l'étanchéité même lorsque l'utilisateur désaxe le piston 3.

Dans le cadre de l'invention, les variantes décrites peuvent être combinées entre elles, au moins de manière partielle.

## Revendications

1. Seringue (1), comprenant :
- un corps (2) délimitant une chambre (C),
- un piston (3) apte à coulisser dans la chambre (C) du corps (2) le long d'un axe longitudinal (X1) de la seringue, le piston comportant une tige (31), une tête (33) et un appuie-doigt (32) qui sont en une seule et même pièce,
- un dispositif de retenue (4) déformable, configuré pour empêcher le piston (3) de sortir entièrement du corps (2), lequel dispositif de retenue :
- est monté en force dans la chambre (C),
- s'étend autour du piston (3) sur un secteur angulaire (A) strictement supérieur à 180° quand il est en position assemblée, et **caractérisé en ce que** ledit dispositif de retenue comporte des nervures externes (46), qui s'étendent en saillie parallèlement à l'axe longitudinal (X1) et qui sont déformables plastiquement lors de l'assemblage du dispositif de retenue dans la chambre.

2. Seringue (1) selon la revendication 1, **caractérisée en ce que** le corps (2) est réalisé en un matériau plastique présentant un module de Young strictement supérieur à 1600 MPa.

3. Seringue (1) selon l'une quelconque des revendications précédentes, **caractérisée en ce que** le dispositif de retenue (4) est monté axialement en force dans la chambre (C).

4. Seringue (1) selon l'une quelconque des revendications précédentes, **caractérisée en ce que** le dispositif de retenue (4) comporte deux parties (41, 42) en forme de C, reliées entre elles par une zone amincie (43) déformable.

5. Seringue (1) selon l'une quelconque des revendications précédentes, **caractérisée en ce que** le dispositif de retenue (4) comporte un passage (P) pour une tige (31) du piston (3) et **en ce que** le diamètre (D33) d'une tête (33) du piston (3) est au moins supérieur de 10% au diamètre (D41) du passage (P) en position assemblée du dispositif de retenue.

6. Seringue (1) selon l'une quelconque des revendications précédentes, **caractérisée en ce que** les nervures externes (46) sont disposées sur le dispositif de retenue (4) en étant réparties à distance les unes des autres autour de l'axe longitudinal (X1).

7. Seringue (1) selon l'une quelconque des revendications précédentes, **caractérisée en ce qu'**au niveau de chacune des nervures externes (46), le dispositif de retenue (4) en position assemblée a une dimension extérieure maximale transversale (D46) qui est strictement supérieure au diamètre (D2) de la chambre (C).

8. Seringue (1) selon l'une quelconque des revendications précédentes, **caractérisée en ce que** le dispositif de retenue (4) comporte au moins un clip externe (48) qui, en position assemblée du dispositif de retenue, est engagé transversalement dans un logement (23) du corps (2) de la seringue (1).

9. Seringue (1) selon l'une quelconque des revendications précédentes, **caractérisée en ce que** le dispositif de retenue (4) comporte un passage (P) pour une tige (31) du piston (3), le passage (P) étant pourvu d'une ouverture (47) d'entrée, et **en ce que**, aussi bien dans une position libre du dispositif de retenue (4) avant assemblage, qu'en position assemblée du dispositif de retenue (4), une largeur (L47) de l'ouverture (47) est inférieure au diamètre (D31) de la tige (31) du piston (3).

10. Seringue (1) selon l'une quelconque des revendications précédentes, **caractérisée en ce que** la tête (33) du piston (3) comporte un élément d'étanchéité (35).

11. Seringue (1) selon la revendication 10, **caractérisée en ce que** la tête (33) du piston (3) comporte une gorge (34) dans laquelle est disposé l'élément d'étanchéité (35).

12. Seringue (1) selon la revendication 10, **caractérisée en ce que** l'élément d'étanchéité (35) fait partie intégrante de la tête (33) du piston (3).

13. Seringue (1) selon l'une quelconque des revendications précédentes, **caractérisée en ce que** le volume de la chambre (C) est inférieur à 25 mL.

14. Procédé d'assemblage d'une seringue (1) selon l'une des revendications précédentes, **caractérisé en ce qu'**il comprend des étapes dans lesquelles :
a) le dispositif de retenue (4) est déformé (F1, F2) pour être monté autour d'une tige (31) du piston (3),
b) le piston (3) est inséré dans la chambre (C) du corps (2),
c) le dispositif de retenue (4) est monté en force dans la chambre (C) du corps (2) du piston (3).

15. Procédé selon la revendication 14, **caractérisée en ce que** dans l'étape c), le dispositif de retenue (4) est assemblé au corps (2) de la seringue (1) par un mouvement de translation (T), coaxialement à l'axe longitudinal (X1).

## Patentansprüche

1. Spritze (1), umfassend:
- einen Körper (2), der eine Kammer (C) begrenzt,
- einen Kolben (3), der in der Kammer (C) des Körpers (2) entlang einer Längsachse (X1) der Spritze gleiten kann, wobei der Kolben eine Stange (31), einen Kopf (33) und eine Fingerauflage (32) aufweist, die aus ein und demselben Stück sind,
- eine deformierbare Haltevorrichtung (4), die dazu ausgebildet ist, den Kolben (3) daran zu hindern, ganz aus dem Körper (2) herauszugehen,
wobei die Haltevorrichtung:
- in der Kammer (C) pressgepasst ist,
- sich über einen Winkelbereich (A) von strikt größer als 180° um den Kolben (3) herum erstreckt, wenn sie in montierter Position ist,
**dadurch gekennzeichnet, dass** die Haltevorrichtung äußere Rippen (46) aufweist, die sich parallel zur Längsachse (X1) vorspringend erstrecken und die bei der Montage der Haltevorrichtung in der Kammer plastisch deformierbar sind.

2. Spritze (1) nach Anspruch 1, **dadurch gekennzeichnet, dass** der Körper (2) aus einem Kunststoffmaterial ausgeführt ist, das einen Elastizitätsmodul von strikt höher als 1600 MPa aufweist.

3. Spritze (1) nach einem der vorhergehenden Ansprüche, **dadurch gekennzeichnet, dass** die Haltevorrichtung (4) axial in die Kammer (C) pressgepasst ist.

4. Spritze (1) nach einem der vorhergehenden Ansprüche, **dadurch gekennzeichnet, dass** die Haltevorrichtung (4) zwei C-förmige Teile (41, 42) aufweist, die durch einen deformierbaren, verjüngten Bereich (43) miteinander verbunden sind.

5. Spritze (1) nach einem der vorhergehenden Ansprüche, **dadurch gekennzeichnet, dass** die Haltevorrichtung (4) einen Durchgang (P) für eine Stange (31) des Kolbens (3) aufweist und dass der Durchmesser (D33) eines Kopfs (33) des Kolbens (3) in der montierten Position der Haltevorrichtung mindestens 10% größer als der Durchmesser (D41) des Durchgangs (P) ist.

6. Spritze (1) nach einem der vorhergehenden Ansprüche, **dadurch gekennzeichnet, dass** die äußeren Rippen (46) so an der Haltevorrichtung (4) angeordnet sind, dass sie voneinander beabstandet um die Längsachse (X1) herum verteilt sind.

7. Spritze (1) nach einem der vorhergehenden Ansprüche, **dadurch gekennzeichnet, dass** die Haltevorrichtung (4) in montierter Position an jeder der äußeren Rippen (46) eine maximale äußere Querabmessung (D46) hat, die strikt größer als der Durchmesser (D2) der Kammer (C) ist.

8. Spritze (1) nach einem der vorhergehenden Ansprüche, **dadurch gekennzeichnet, dass** die Haltevorrichtung (4) mindestens einen äußeren Clip (48) aufweist, der in montierter Position der Haltevorrichtung in einer Aufnahme (23) des Körpers (2) der Spritze (1) quer in Eingriff steht.

9. Spritze (1) nach einem der vorhergehenden Ansprüche, **dadurch gekennzeichnet, dass** die Haltevorrichtung (4) einen Durchgang (P) für eine Stange (31) des Kolbens (3) aufweist, wobei der Durchgang (P) mit einer Eingangsöffnung (47) versehen ist, und dass eine Breite (L47) der Öffnung (47) sowohl in einer freien Position der Haltevorrichtung (4) vor der Montage als auch in montierter Position der Haltevorrichtung (4) kleiner als der Durchmesser (D31) der Stange (31) des Kolbens (3) ist.

10. Spritze (1) nach einem der vorhergehenden Ansprüche, **dadurch gekennzeichnet, dass** der Kopf (33) des Kolbens (3) ein Dichtelement (35) aufweist.

11. Spritze (1) nach Anspruch 10, **dadurch gekennzeichnet, dass** der Kopf (33) des Kolbens (3) eine Kehle (34) aufweist, in der das Dichtelement (35) angeordnet ist.

12. Spritze (1) nach Anspruch 10, **dadurch gekennzeichnet, dass** das Dichtelement (35) ein integrierter Teil des Kopfs (33) des Kolbens (3) ist.

13. Spritze (1) nach einem der vorhergehenden Ansprüche, **dadurch gekennzeichnet, dass** das Volumen der Kammer (C) kleiner als 25 ml ist.

14. Verfahren zum Zusammenbau einer Spritze (1) nach einem der vorhergehenden Ansprüche, **dadurch gekennzeichnet, dass** es Schritte umfasst, bei denen:
a) die Haltevorrichtung (4) zum Montieren um eine Stange (31) des Kolbens (3) herum deformiert (F1, F2) wird,
b) der Kolben (3) in die Kammer (C) des Körpers (2) eingeführt wird,
c) die Haltevorrichtung (4) in die Kammer (C) des Körpers (2) des Kolbens (3) pressgepasst wird.

15. Verfahren nach Anspruch 14, **dadurch gekennzeichnet, dass** die Haltevorrichtung (4) in Schritt c) durch eine koaxial zu der Längsachse (X1) verlaufende Translationsbewegung (T) im Körper (2) der Spritze (1) montiert wird.

## Claims

1. Syringe (1) comprising:
- a body (2) delimiting a chamber (C),
- a piston (3) able to slide in the chamber (C) of the body (2) along a longitudinal axis (X1) of the syringe, the piston including a rod (31), a head (33) and a finger rest (32) which are all in one piece,
- a deformable retention device (4) designed to prevent the piston (3) from leaving the body (2) entirely, which retention device:
- is force-fitted in the chamber (C),
- extends around the piston (3) over an angular sector (A) strictly greater than 180° when it is in the assembled position, and
**characterized in that** said retention device has protruding external ribs (46) which extend parallel to the longitudinal axis (X1) and are plastically deformable when the retention device is assembled in the chamber.

2. Syringe (1) according to Claim 1, **characterized in that** the body (2) is made of a plastic material having a Young's modulus strictly greater than 1600 MPa.

3. Syringe (1) according to either one of the preceding claims, **characterized in that** the retention device (4) is force-fitted axially in the chamber (C).

4. Syringe (1) according to any one of the preceding claims, **characterized in that** the retention device (4) includes two C-shaped parts (41, 42) connected to each other by a deformable thinned area (43).

5. Syringe (1) according to any one of the preceding claims, **characterized in that** the retention device (4) includes a passage (P) for a rod (31) of the piston (3) and **in that** the diameter (D33) of a head (33) of the piston (3) is at least 10% greater than the diameter (D41) of the passage (P) in the assembled position of the retention device.

6. Syringe (1) according to any one of the preceding claims, **characterized in that** the external ribs (46) are disposed on the retaining device (4) at a distance from one another around the longitudinal axis (X1).

7. Syringe (1) according to any one of the preceding claims, **characterized in that** at the level of each of the external ribs (46) the retention device (4) in the assembled position has a maximum outside transverse dimension (D46) that is strictly greater than the diameter (D2) of the chamber (C).

8. Syringe (1) according to any one of the preceding claims, **characterized in that** the retention device (4) includes at least one external clip (48) which in the assembled position of the retention device is engaged transversely in a housing (23) of the body (2) of the syringe (1).

9. Syringe (1) according to any one of the preceding claims, **characterized in that** the retention device (4) includes a passage (P) for a rod (31) of the piston (3), the passage (P) including an entry opening (47), and **in that**, both in a free position of the retention device (4) before assembly and in the assembled position of the retention device (4), a width (L47) of the opening (47) is less than the diameter (D31) of the rod (31) of the piston (3).

10. Syringe (1) according to any one of the preceding claims, **characterized in that** the head (33) of the piston (3) includes a sealing element (35).

11. Syringe (1) according to Claim 10, **characterized in that** the head (33) of the piston (3) includes a groove (34) in which the sealing element (35) is disposed.

12. Syringe (1) according to Claim 10, **characterized in that** the sealing element (35) is an integral part of the head (33) of the piston (3).

13. Syringe (1) according to any one of the preceding claims, **characterized in that** the volume of the chamber (C) is less than 25 mL.

14. Method of assembling a syringe (1) according to any one of the preceding claims, **characterized in that** it comprises steps in which:
a) the retention device (4) is deformed (F1, F2) to be mounted around a rod (31) of the piston (3),
b) the piston (3) is inserted in the chamber (C) of the body (2),
c) the retention device (4) is force-fitted in the chamber (C) of the body (2) of the piston (3).

15. Method according to Claim 14, **characterized in that** in step c) the retention device (4) is assembled to the body (2) of the syringe (1) by a translation movement (T), coaxially with the longitudinal axis (X1).
